(19) Europäisches Patentamt
European Patent Office
Office européen des brevets

(11) Veröffentlichungsnummer : **0 054 685**
**B1**

(12) **EUROPÄISCHE PATENTSCHRIFT**

(45) Veröffentlichungstag der Patentschrift : **09.07.86**

(51) Int. Cl.⁴ : **C 08 F 2/22**, C 08 F220/32, C 12 N 11/08, G 01 N 33/531, G 01 N 33/74

(21) Anmeldenummer : **81108886.3**

(22) Anmeldetag : **24.10.81**

(54) **Hydrophile Latexpartikel, Verfahren zu deren Herstellung und deren Verwendung.**

(30) Priorität : **23.12.80 DE 3048883**
**23.12.80 CS 9235/80**

(43) Veröffentlichungstag der Anmeldung : **30.06.82 Patentblatt 82/26**

(45) Bekanntmachung des Hinweises auf die Patenterteilung : **09.07.86 Patentblatt 86/28**

(84) Benannte Vertragsstaaten :
**AT BE CH DE FR GB IT LI LU NL SE**

(56) Entgegenhaltungen :
**EP-A- 0 007 042**
**DE-A- 2 203 377**
**DE-A- 2 812 845**
**FR-A- 836 967**
**GB-A- 636 385**
**US-A- 4 016 133**
**US-A- 4 138 383**
**US-A- 4 210 723**
**Die Akte enthält technische Angaben, die nach dem Eingang der Anmeldung eingereicht wurden und die nicht in dieser Patentschrift enthalten sind.**

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

**Tschecoslowakische Akademie der Wissenschaften**
**Narodni 3**
**CS-111 42 Prag (CS)**

(72) Erfinder : **Batz, Hans-Georg, Dr.rer.nat.**
**Traubinger-Strasse 63**
**D-8132 Tutzing (DE)**
Erfinder : **Tanswell, Paul, Dr.phil.**
**Rudolfstrasse 112**
**D-8033 Planegg (DE)**
Erfinder : **Baier, Manfred, Dr.rer.nat.**
**Kurt-Stieler-Strasse 1**
**D-8134 Pöcking-Possenhofen (DE)**
Erfinder : **Bouchal, Karel, Dr.Ing.**
**Belojanisova 6**
**150 00 Prag 5 (CS)**
Erfinder : **Kalal, Jaroslav, Prof.Dr.Ing.**
**Belohorska 135**
**160 00 Prag 6 (CS)**
Erfinder : **Svec, Frantisek, Dr.Ing.**
**Pricna 26**
**273 43 Hrebec (CS)**
Erfinder : **Zurkova geb.Hrubá, Eva, Dr.Ing.**
**Jeremenkova 62**
**147 00 Prag 4 (CS)**

(74) Vertreter : **Daum, Martin, Dr. et al**
**Boehringer Mannheim GmbH Patentabteilung Postfach 31 01 20**
**D-6800 Mannheim 31 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 054 685 B1

**Beschreibung**

Die Erfindung betrifft hydrophile Latexpartikel, ein Verfahren zu deren Herstellung und deren Verwendung als Trägermaterial für biologisch und/oder immunologisch aktive Substanzen in diagnostischen Mitteln.

Für die Agglutination von Antigen-Antikörper-Komplexen, die in der Immunologie im Rahmen vieler diagnostischer Bestimmungen ausgenutzt wird, weil sie besonders schnell und einfach durchgeführt und häufig mit bloßem Auge beobachtet werden kann, verwendet man seit langem hydrophobe Latexpartikel als Träger von immunologisch aktiven Substanzen, beispielsweise von Antikörpern. Diese hydrophoben Latexpartikel bestehen meist aus Polystyrolhomo- oder Copolymerisaten, beispielsweise Styrol-Butadien-Copolymerisaten oder Acrylnitril-Butadien-Styrol-Copolymerisaten (ABS) und werden durch Emulsionspolymerisation hergestellt.

Bei der seit langem bekannten Emulsionspolymerisation sind im allgemeinen vier Komponenten zugegen : ein in Wasser schwerlösliches Monomer oder ein Gemisch verschiedener in Wasser schwerlöslicher Monomerer, Wasser, ein Emulgator und ein wasserlöslicher Initiator. Das Monomere wird dabei durch den Emulgator in Form feiner Tröpfchen emulgiert, wobei sich durch Zusammenlagerung von mehreren Emulgatormolekülen u. a. größere Micellen bilden, die teilweise leer und teilweise mit Monomermolekülen gefüllt sind ; letzteres wird als « Solubilisierung » des Monomeren bezeichnet. Der wasserlösliche Initiator bildet Radikale, die die Polymerisation sowohl von einzelnen Monomermolekülen in der Wasserphase als auch in den monomergefüllten Micellen sowie in den Monomertröpfchen auslösen bzw. aktivieren können. Tatsächlich findet aber die Polymerisation überwiegend in den gequollenen Micellen statt, da einerseits die Monomerkonzentration in den Micellen wesentlich größer ist als in der Umgebung einzelner gelöster Monomermoleküle und andererseits die Wahrscheinlichkeit der Aktivierung von Micellen wegen ihrer im Vergleich zur Zahl der Monomertröpfchen wesentlich größeren Anzahl beträchtlich höher ist. Der Durchmesser der gefüllten Micellen nimmt während der Polymerisation zu, bis diese schließlich in kugelförmige Latexteilchen übergehen. Die Emulgatormoleküle der nichtaktivierten Micellen und diejenigen aus der Oberfläche der verbrauchten Monomertröpfchen bedecken die Oberfläche der Latexteilchen und tragen so zur Stabilisierung der entstehenden Polymerdispersion bei.

Diese nach dem bekannten Verfahren unter Zusatz eines Emulgators oder Emulsionsstabilisators, welcher beispielsweise ein Tensid oder Netzmittel sein kann, hergestellten Latices haben folgende Nachteile, die insbesondere ihre Verwendung als Träger von immunologisch aktiven Substanzen stören und ihren Einsatz in kontinuierlichen Lösungsmeßsystemen verhindern :

1. An der hydrophoben Oberfläche der Latexpartikel werden neben den gewünschten immunologisch aktiven Substanzen, beispielsweise Antikörpern, unspezifisch eine Vielzahl anderer Serumbestandteile gebunden ;

2. die nur adsorptiv, nicht kovalent gebundenen immunologisch aktiven Substanzen können sich während der Messung im Verlauf eines diagnostischen Tests wieder ablösen ;

3. die bei der Emulsionspolymerisation als Emulgator oder Stabilisator verwendeten Tenside können die Struktur und damit die Aktivität der biologisch aktiven Proteine zerstören, weil sie in die wäßrige Lösung diffundieren ;

4. beim Entfernen der sie stabilisierenden Tenside koaguliert die Latexsuspension und auch beim Zentrifugieren wird die Stabilisierung gebrochen. Der hierbei entstehende Niederschlag kann nur schwer oder überhaupt nicht mehr zum vorherigen Zustand resuspendiert werden.

Zur Vermeidung dieser Nachteile wurden bereits verschiedene Vorschläge gemacht, die aber immer nur einen Teil der vorstehend genannten Probleme lösen konnten :

So sind aus der DE-AS 2 203 377 hydrophobe Latexpartikel mit einer Teilchengröße von 0,01 bis 0,9 µm aus carboxylierten ABS-Copolymeren und carboxylierten Styrol-Butadien-Copolymeren bekannt, die als serologisch inerte Träger für biologisch aktive Proteine verwendet werden können, wobei die Proteine kovalent an den Träger gebunden werden, und zwar über die in den Latex eingeführten Carboxylgruppen, unter Ausbildung von Amidbindungen.

Aus der DE-OS 2 812 845 sind hydrophobe Latices mit einer Teilchengröße von 0,05 bis 1 µm aus ABS-Copolymeren bekannt, bei denen der Latex ebenfalls mit Carboxylgruppen modifiziert und mit einer reaktiven Seitenkette kondensiert ist, so daß immunologisch aktive Substanzen ebenfalls kovalent gebunden werden können.

Mit diesen bekannten hydrophoben Latices wird zwar das Problem Nr. 2 (s. o.) gelöst, alle übrigen Nachteile bleiben aber unverändert bestehen.

Man hat deshalb auch schon versucht, anstelle hydrophober Latices hydrophile Gele als Träger für immunologisch aktive Substanzen zu verwenden. Da hydrophile Gele keine oder nur sehr geringe Adsorptionseigenschaften haben, andererseits aber die kovalente Bindung von Proteinen an solche Gele bekannt ist, hat man « Mikrogele » vorgeschlagen, die aufgrund ihrer Herstellungsweise und ihres Teilchendurchmessers ebensogut als « Latices » bezeichnet werden könnten. Solche hydrophilen Latices sind beispielsweise aus der US-PS 4 138 383 bekannt. Sie bestehen aus kugelförmigen Teilchen mit einem Durchmesser von weniger als 0,35 µm, die unter den Bedingungen einer durch freie Radikale initiierten wäßrigen Emulsionspolymerisation hergestellt werden, wobei als Monomere Acrylamide, Acrylsäure, Methacrylsäure oder Acrylate verwendet werden. Als Emulgatoren werden beispielsweise

Metallseifen verwendet. An die so erhaltenen hydrophilen Mikrogele werden biologisch und/oder immunologisch aktive Substanzen in an sich bekannter Weise über Carbodiimid- oder Glutardialdehyd-Brücken kovalent gebunden. Damit sind zwar die Probleme Nr. 1 und 2 (s. o.) gelöst worden, nicht aber die Probleme Nr. 3 und 4, da die Emulsionspolymerisation nach wie vor unter Zusatz eines Emulgators oder Stabilisators durchgeführt werden muß.

In der EP-A 0 007 042 werden Kautschuk-Latices beschrieben, die ohne Emulgator aus (Meth)Acrylsäure und einem Gemisch aus acyclischen konjugierten Dienen und Arylvinylmonomeren und/oder (Meth)Acrylnitril hergestellt werden. Die Herstellung dieser Latices ohne Emulgator gelingt nur durch einen komplizierten Mehrstufenmechanismus bei einem bestimmten pH-Wert und führt ebenfalls nur zu hydrophoben Endprodukten.

Polystyrolkügelchen, die mit einer freie Epoxy-Gruppen enthaltenden Schicht überzogen sind, werden in dem USP 4,210,723 erwähnt. Die Schicht, welche die freien Epoxygruppen enthält, wird auf den Polystyrolkügelchen durch übliche Polymerisation unter Verwendung von Detergentien erzeugt. Es treten die bekannten Probleme auf, daß die Detergentien nicht vollständig entfernt werden können und somit Restkonzentrationen an Detergentien in den Endprodukten vorhanden sind, welche deren Anwendbarkeit als Träger für biologisch aktive Materialien beeinträchtigen.

Der Erfindung liegt die Aufgabe zugrunde, hydrophile Latexpartikel, ein Verfahren zu deren Herstellung und ein diese enthaltendes diagnostisches Mittel zu schaffen, mit denen es gelingt, alle vier oben genannten Nachteile zu vermeiden. Der Erfindung liegt also insbesondere die Aufgabe zugrunde, hydrophile Latexpartikel zu schaffen, die in der Lage sind, biologisch und/oder immunologisch aktive Substanzen kovalent zu binden, die die Struktur und damit die Aktivität der biologisch aktiven Proteine nicht beeinträchtigen, deren Stabilisierung beim Zentrifugieren nicht gebrochen wird und die sich koagulieren und anschließend wieder leicht resuspendieren lassen.

Diese Aufgabe wird gemäß der Erfindung durch aus einem Homo- oder Copolymerisat von in Wasser schwerlöslichen Monomeren bestehende hydrophile Latexpartikel gelöst, die dadurch gekennzeichnet sind, daß sie durch Emulsionspolymerisation in Anwesenheit eines wasserlöslichen, Radikale bildenden Initiators, jedoch ohne jeden Zusatz eines Emulgators, Stabilisators oder Netzmittels herstellbar sind, und daß mindestens ein Teil der Monomeren, aus denen die hydrophilen Latexpartikel aufgebaut sind, ein mindestens eine polymerisierbare C = C-Doppelbindung im Molekül enthaltendes Epoxid ist.

Es hat sich überraschenderweise gezeigt, daß entgegen der in Fachkreisen seit langem herrschenden Meinung, die auch in dem eingangs zitierten Stand der Technik zum Ausdruck gebracht wird, der Zusatz eines Emulgators, Stabilisators oder eines Netzmittels zur Durchführung der Emulsionspolymerisation gar nicht erforderlich ist. Damit entfällt aber die meist sehr schwierige und umständliche Entfernung von Emulgatorresten aus den polymeren Latexpartikeln, die bisher zwingend erforderlich war, weil die als Emulgatoren verwendeten Metallseifen oder Tenside aus den Latexpartikeln diffundierten und die biologische Aktivität der an die Latexpartikel kovalent gebundenen Proteine beeinträchtigten oder vollkommen zerstörten.

Als besonders vorteilhaft hat sich die Verwendung von mindestens eine polymerisierbare Doppelbindung enthaltenden Glycidyl-Verbindungen als Monomer erwiesen. Die Struktur dieser Verbindungen weist in der polymerisierbaren Doppelbindung einen hydrophoben und in der Epoxid-Gruppe, dem Oxiran-Ring, zugleich einen hydrophilen Teil auf. Die erfindungsgemäß entstehenden Latexsuspensionen koagulieren nicht trotz Abwesenheit jeglichen Emulgators, Stabilisators oder Netzmittels. Die Stabilisierung der Latexsuspension wird auch beim Zentrifugieren nicht gebrochen. Da die endständigen Epoxidgruppen für verschiedene Umsetzungen (Hydrolyse, Ammonolyse, Aminolyse, Kondensationen) sehr leicht zugänglich sind, müssen die Epoxidgruppen die Oberfläche der monodispers verteilten Latexkügelchen so bedecken, daß sie in die Wasserphase hinaus orientiert sind.

Als Monomere werden erfindungsgemäß vorzugsweise Glycidylmethacrylat, Glycidylacrylat, Glycidylvinyläther, Glycidylvinylphthalat und 3,4-Epoxybut (1) en verwendet. Es kann dabei ausschließlich eines dieser Monomere verwendet werden, so daß die entstehenden hydrophilen Latexpartikel ein Homopolymerisat darstellen, es kann aber auch ein Gemisch dieser Monomere copolymerisiert werden.

Zur Steuerung des Gehaltes an Epoxidgruppen können mit einer oder mehreren der genannten Glycidylverbindungen auch andere Monomere copolymerisiert werden, beispielsweise Styrol, Diene, Acrylamide, Methacrylamide, Alkyl-, Hydroxyalkyl- und Aminoalkylacrylate, Alkyl-, Hydroxyalkyl- und Aminoalkylmethacrylate, Vinyläther, Vinylester, und N-Vinylpyrrolidon.

Es kann auch in Gegenwart monomerer, polymerisierbarer Derivate von Farbstoffen oder fluoreszierenden Verbindungen, z. B. Fluorescein, polymerisiert werden. Auf diese Weise werden farbige bzw. fluoreszierende Latexpartikel erhalten, die sich zum Nachweis von Antigenen bzw. Antikörpern in menschlichen oder tierischen Geweben eignen. Diese Nachweismethode eignet sich besonders vorteilhaft zur Herstellung von Gewebeschnitten in der Histologie. Als monomere, polymerisierbare Derivate werden vorzugsweise Farbstoffe bzw. fluoreszierende Verbindungen eingesetzt, in die in an sich bekannter Weise Methacryl- bzw. Acryl-Reste eingebaut worden sind.

Um die Schwerlöslichkeit der entstehenden Latexpartikel in Wasser zu erhöhen, können während der Emulsionspolymerisation übliche Vernetzungsmittel zugesetzt werden, beispielsweise Alkylen- oder Hydroxyalkylendiacrylate, Alkylen- oder Hydroxyalkylendimethacrylate, Alkylenbisacrylamide oder Alkylenbismethacrylamide und Divinylbenzol.

Als Initiator kann jeder üblicherweise für die Emulsionspolymerisation verwendete wasserlösliche Initiator verwendet werden; erfindungsgemäß werden vorzugsweise Peroxodisulfate, Peroxoborate, Wasserstoffperoxid oder geeignete Redoxsysteme eingesetzt.

Das erfindungsgemäße Verfahren der emulgatorfreien Emulsionspolymerisation von einem oder verschiedenen in Wasser schwerlöslichen Monomeren zur Herstellung der hydrophilen Latexpartikel ist gegenüber Luftsauerstoff bzw. freiem Sauerstoff überhaupt empfindlich. Der Sauerstoff muß deshalb sehr sorgfältig aus allen Polymerisationskomponenten und Gefäßen durch gründliches Auskochen, durch Destillation unter Inertgasatmosphäre oder durch Hindurchleiten von Stickstoff, Argon oder einem anderen Inertgas entfernt werden.

Die emulgatorfreie Emulsionspolymerisation wird erfindungsgemäß vorzugsweise mit einem Flottenverhältnis, bezogen auf die Volumina, zwischen Wasser- und Monomerenphase von 8 : 1 bis 16 : 1 durchgeführt.

Die Konzentration des in der wäßrigen Phase gelösten Initiators beträgt vorzugsweise zwischen 0,5 und 1,5 g/l und die Konzentration des Epoxids in der Monomerenphase beträgt vorzugsweise 1 bis 100 Gew.%.

Die Emulsionspolymerisation wird vorzugsweise bei einer Temperatur von 0 bis 80 °C durchgeführt. Die Temperatur ist abhängig vom gewählten Initiator: bei Verwendung von Kaliumperoxodisulfat arbeitet man vorzugsweise bei 60 bis 70 °C. Ebenfalls abhängig von der Wahl des Initiators ist die Reaktionsdauer, die zwischen 5 und 40 Stunden beträgt.

Die erfindungsgemäßen hydrophilen Latexpartikel sind streng kugelförmige, monodispers verteilte und untereinander annähernd gleich große Teilchen mit einem Durchmesser von etwa 0,15 bis 1,5 μm.

Die hydrophilen Latexpartikel können nach beendeter Emulsionspolymerisation noch Reste nichtauspolymerisierter Monomerer enthalten, die durch Wasserdampfdestillation oder Dialyse beseitigt werden können. Auch hierbei kommt die besonders vorteilhafte Eigenschaft der erfindungsgemäßen hydrophilen Latexpartikel zum Tragen, die darin besteht, daß die Latexpartikel durch Zentrifugieren sedimentiert werden können, ohne daß die Stabilisierung gebrochen wird, so daß sie anschließend wieder redispergiert werden können. Auf diese Weise kann der erfindungsgemäße Latex durch mehrfaches Zentrifugieren und Dekantieren auf einfache Weise gereinigt werden.

Die endständigen freien Epoxidgruppen des erfindungsgemäßen Latex sind gegenüber den unterschiedlichsten chemischen Substanzen hoch reaktionsfähig und können deshalb leicht hydrolysiert, mit Periodat oder Periodsäure zur Aldehydgruppe oxidiert, mit Ammoniak, primären Aminen, Diaminen oder Hydrazinen zu prim. od. sek. Aminogruppen umgesetzt oder mit Hilfe anderer bekannter Reaktionen modifiziert werden. Die Emulsion des erfindungsgemäßen Latex weist trotz Fehlens eines Emulgators eine derart hohe Stabilität auf, daß die Modifizierung der Epoxidgruppen gewünschtenfalls auch schon während der Emulsionspolymerisation durchgeführt werden kann, so daß bereits aus der Polymerisationsreaktion ein Latex entsteht, der an der Oberfläche beispielsweise mit primären Aminogruppen modifiziert ist. Die modifizierten oder derivatisierten Epoxidgruppen stehen dann für die « Kupplung » mit biologisch und/oder immunologisch aktiven Proteinen, die somit kovalent an die als Träger fungierenden hydrophilen Latexpartikel gebunden werden, zur Verfügung.

Die eingangs genannte Aufgabe wird somit weiter durch die Verwendung der erfindungsgemäßen hydrophilen Latexpartikel als serologisch inerte Träger für biologisch und/oder immunologisch aktive Substanzen gelöst, beispielsweise als Träger für Peptide, Proteine, Enzyme, Hormone, Vitamine, Antigene, Antikörper und Mikroorganismen.

Gegenstand der Erfindung ist somit weiterhin ein diagnostisches Mittel, enthaltend erfindungsgemäße hydrophile Latexpartikel als Träger und an diesen Träger direkt oder über ein Kupplungsmittel als « Brücke » kovalent gebundene biologisch und/oder immunologisch aktive Substanzen der vorstehend genannten Art.

Die erfindungsgemäßen diagnostischen Mittel eignen sich besonders zum Einsatz in Radioimmuno- (RIA), Enzymimmuno- (EIA) und sogenannten ELISA (enzymelinked immunosorbent assay)-Tests.

## Beispiel 1

In 80 ml destilliertem Wasser werden 0,08 g Kaliumperoxodisulfat ($K_2S_2O_8$) gelöst und dadurch von Luftsauerstoff befreit, daß 30 Minuten lang Stickstoff hindurchgeleitet wird. Gleichzeitig werden 10 ml Glycidylmethacrylat auf dieselbe Weise von Luftsauerstoff befreit. Beide Komponenten werden in einen Glasreaktor verbracht und weitere 10 Minuten lang mit Stickstoff behandelt. Danach wird der Reaktor geschlossen und die Reaktion unter ständigem Rühren 6 Stunden lang bei einer Temperatur von 65 °C durchgeführt. Nach dieser Zeit beträgt der Umsatz 98 %. Das Reaktionsprodukt ist ein Latex aus kugelförmigen, monodispers verteilten Polyglycidylmethacrylat-Partikeln mit einem Durchmesser von 0,44 μm.

## Beispiel 2

Wie in Beispiel 1 beschrieben, werden 160 ml destilliertes Wasser, in dem 0,08 g $K_2S_2O_8$ gelöst sind, und 10 ml eines Gemischs aus 15 Gew.% Glycidylmethacrylat und 85 Gew.% Styrol getrennt vom Sauerstoff

befreit und 6 Stunden lang unter ständigem Rühren bei einer Temperatur von 65 °C miteinander umgesetzt. Danach beträgt der Umsatz 71,6 %. Die nichtauspolymerisierten Restmonomeren werden durch Wasserdampfdestillation beseitigt. Die entstehenden monodispersen copolymeren Latexpartikel haben einen Durchmesser von 0,22 µm.

## Beispiel 3

Wie in Beispiel 1 beschrieben, werden eine Lösung von 0,1 g $K_2S_2O_8$ in 100 ml destilliertem Wasser und 10 ml eines Gemischs aus 15 Gew.% Glycidylmethacrylat und 85 Gew.% Vinylacetat miteinander umgesetzt. Der Umsatz beträgt 80 %. Die Restmonomeren werden durch Wasserdampfdestillation entfernt. Der entstehende Latex besteht aus monodispersen, kugelförmigen Teilchen mit einem Durchmesser von 0,16 µm.

100 ml des so hergestellten Latex werden mit 100 ml 0,1 M-NaOH-Lösung vermischt und bei einer Temperatur von etwa 25 °C 24 Stunden stehengelassen. Auch während und nach der Hydrolyse bleibt die Stabilität des Latex erhalten. Die Emulsion wird zentrifugiert, der Überstand abgegossen und die feste Phase wieder in Wasser redispergiert. Das Zentrifugieren und Redispergieren wird zweimal wiederholt. Die entstehende neutrale Emulsion wird mit 1 M-$H_2SO_4$ auf einen pH-Wert von 3 gebracht und mit Periodsäure in einer den Epoxidgruppen äquivalenten Menge versetzt. Die Oxidation wird bei 25 °C etwa 24 Stunden lang durchgeführt ; anschließend werden die nichtumgesetzten niedermolekularen Stoffe durch Dialyse entfernt. Die modifizierten Latexpartikel der stabilen Emulsion enthalten 2,8 Gew.% oder 0,97 mMol/g an Aldehydgruppen.

## Beispiel 4

Wie in Beispiel 1 beschrieben, werden eine Lösung von 0,1 g $K_2S_2O_8$ in 100 ml destilliertem Wasser und 10 ml eines Gemischs aus 15 Gew.% Glycidylmethacrylat und 85 Gew.% Isopren bei einer Temperatur von 65 °C 24 Stunden lang umgesetzt. Der Umsatz beträgt 76 %. Die Restmonomeren werden anschließend durch Wasserdampfdestillation entfernt, wobei stabile, monodispers verteilte kugelförmige Latexpartikel mit einem Durchmesser von 0,25 µm entstehen.

100 ml dieser Emulsion werden anschließend mit 100 ml wäßriger Ammoniaklösung (25 %ig) versetzt und 24 Stunden lang bei Zimmertemperatur stehengelassen, wobei die Epoxidgruppen durch Ammonolyse in Aminogruppen übergehen. Das Reaktionsprodukt wird anschließend mit Periodsäure behandelt, wobei ein Latex entsteht, der 4,5 Gew.% oder 1,55 mMol/g an Aldehydgruppen enthält.

## Beispiel 5

Wie in Beispiel 1 beschrieben, werden eine Lösung von 1,5 g $K_2S_2O_8$ in 1,5 l Wasser und 15 ml Glycidylmethacrylat getrennt von Sauerstoff befreit und miteinander umgesetzt. Weitere 120 ml von sauerstoffreiem Glycidylmethacrylat werden kontinuierlich während 6 Stunden unter Sauerstoffausschluß in das Reaktionsgefäß zugetropft. Danach wird die Polymerisation noch 30 Minuten lang fortgesetzt. Der Umsatz beträgt dann 85 %, und die entstehenden Partikel haben einen Durchmesser von 1,1 µm.

## Beispiel 6

Wie in Beispiel 1 beschrieben, werden 10 ml eines Gemischs von 1 Gew.% Glycidylmethacrylat und 99 Gew.% Styrol und eine Lösung von 0,1 g $K_2S_2O_8$ in 100 ml destilliertem Wasser bei 65 °C während 22 Stunden polymerisiert, wobei hydrophile Latexpartikel mit einem Durchmesser von 0,5 µm entstehen. Der Umsatz beträgt 90 %.

## Beispiel 7

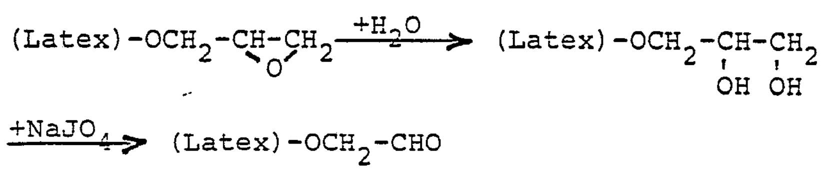


20 ml einer nach Beispiel 1 hergestellten Latex-Suspension werden mit 5 ml 2N Natronlauge über Nacht bei Raumtemperatur gerührt und dann 5 Std. gegen Wasser dialysiert. Die verbleibende Suspension wird nach Zusatz von 100 mg Natriumperjodat auf pH 3 eingestellt und über Nacht bei Raumtemperatur gerührt und dann 4 Std. gegen dest. Wasser dialysiert.

Beispiel 8

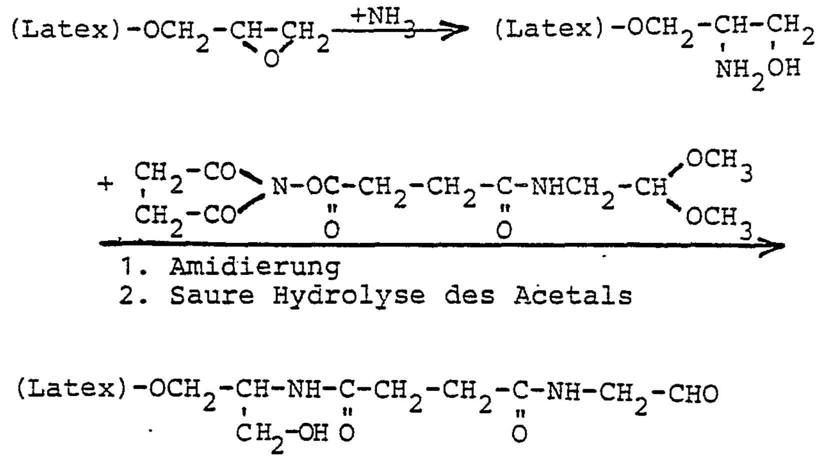


20 ml einer nach Beispiel 1 hergestellten Latex-Suspension werden mit 10 ml konzentrierter Ammoniak-Lösung 20 Std. bei Raumtemperatur gerührt und 48 Std. gegen fließendes Wasser dialysiert. Die erhaltene Suspension wird abzentrifugiert. Der Stickstoff-Gehalt der Trockensubstanz liegt danach bei etwa 1 %, was einer Aminierung von etwa jeder 10. Epoxid-Einheit entspricht, bei einer bevorzugten Reaktion an der Oberfläche aber sicher einem höheren Derivatisierungsgrad entspricht. Die abzentrifugierte Festsubstanz wird mit 20 ml 0,1 N Natronlauge wieder aufgenommen. Dazu werden unter Rühren 1,5 g Bernsteinsäurehydroxysuccinimid-ester-amidoacetaldehyd-acetal (gelöst in 6 ml Dimethylformamid) getropft. Die entstehende Suspension wird noch 3 Std. gerührt, danach mit 1N Salzsäure auf pH 3 gebracht und dann 12 Std. gegen fließendes dest. Wasser dialysiert.

Beispiel 9

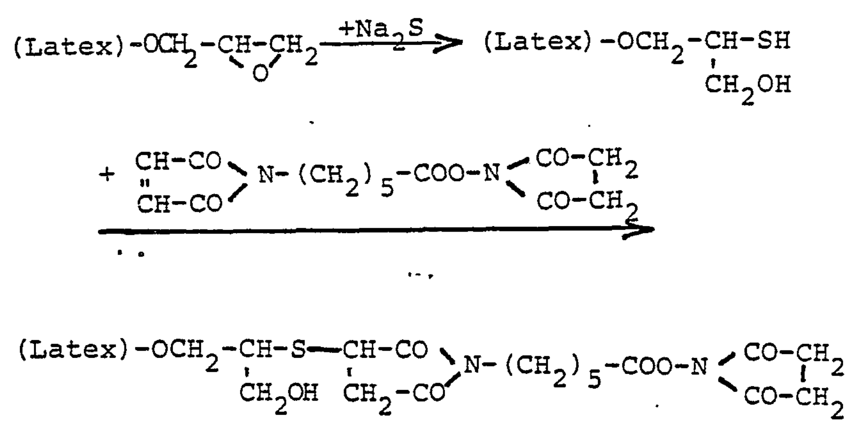


20 ml einer nach Beispiel 1 hergestellten Latex-Suspension werden mit 0,5 g $Na_2S \cdot 9H_2O$ versetzt und 2 Tage bei Raumtemperatur gerührt. Anschließend wird gegen fließendes dest. Wasser dialysiert, bis die Suspension geruchsfrei ist, dann wird die Suspension abzentrifugiert. Die Trockensubstanz enthält etwa 2 % Schwefel, was einer Derivatisierung etwa jeder 10. Epoxid-Einhet entspricht, bei einer bevorzugten Reaktion an der Oberfläche aber sicher einem höheren Derivatisierungsgrad entspricht.

Das abzentrifugierte Produkt wird mit 1 g 6-Maleinimido-hexansäure-hydroxysuccinimidester (gelöst in 6 ml Dimethylformamid) versetzt,3 Std. bei Raumtemperatur gerührt und dann 12 Std. gegen fließendes dest. Wasser dialysiert.

Beispiel 10

Bildung von Latex-gamma-Globulin-Konjugaten

1 ml gamma-Globulin-Lösung (enthaltend 56,6 mg Protein) werden mit jeweils 20 ml einer
a) nach Beispiel 1 hergestellten,
b) nach Beispiel 7,
c) nach Beispiel 8 und
d) nach Beispiel 9 derivatisierten

Latex-Suspension versetzt und 12 Std. bei Raumtemperatur dialysiert. Die verbleibenden Suspensionen werden abzentrifugiert. Anschließend wird im Überstand freies Protein bestimmt. Aus dem gefundenen Wert läßt sich der an die Latex-Teilchen gebundene Anteil berechnen. Danach enthält :

| | |
|---|---|
| Ansatz a | 8 mg gamma-Globulin, gebunden |
| Ansatz b | 43 mg gamma-Globulin, gebunden |
| Ansatz c | 15 mg gamma-Globulin, gebunden |
| Ansatz d | 19 mg gamma-Globulin, gebunden |

Beispiel 11

Bildung von Latex-IgG-Konjugaten

Wie in Beispiel 10 beschrieben werden aus einer IgG-Lösung und verschiedenen Latex-Suspensionen Latex-IgG-Konjugate hergestellt. Durch Antikörper-Komplexbildung wird die Beladung der Latex-Partikel mit IgG-Molekülen nachgewiesen.

Im folgenden werden zwei diagnostische Mittel als Beispiele für die Verwendung der erfindungsgemäßen hydrophilen Latexpartikel beschrieben, und zwar zur immunologischen Bestimmung von Thyroxin ($T_4$) mit Hilfe von $T_4$-Antikörpern aus Anti-$T_4$-Serum vom Schaf und zur Bestimmung von Humanthyr(e)otropin (TSH) im Serum mit Hilfe der Doppelantikörper-Trenntechnik.

Beispiel 12

Verwendung von Homopolyglycidylmethacrylat-Latexpartikeln als Träger für Anti-$T_4$-Serum vom Schaf ; diagnostisches Mittel zur Durchführung eines $T_4$-ELISA

Für die Bestimmung von Thyroxin ($T_4$) mittels ELISA wurden zunächst $T_4$-Antikörper aus einem Anti-$T_4$-Serum vom Schaf an die erfindungsgemäßen hydrophilen Homopolyglycidylmethacrylat-Latexpartikel kovalent gebunden, indem letztere nach einem der Beispiele 7-9 derivatisiert und in Analogie zu den Beispielen 10 und 11 mit den $T_4$-Antikörpern umgesetzt werden. Die so erhaltenen solid-face-Antikörper stellen das erste Reagens für den Test dar. Als zweites Reagens wird $T_4$ in an sich bekannter Weise mit einem hierfür geeigneten Enzym, beispielsweise Peroxidase (POD) oder β-Galaktosidase (β-Gal), markiert, d. h. zu einem Enzymkonjugat « gekoppelt ». Als Enzym wird im vorliegenden Beispiel β-Gal verwendet. Das dritte Reagens ist die einen unbekannten Gehalt an $T_4$ aufweisende Probe und das vierte Reagens ein übliches Substrat für die β-Gal des Enzymkonjugats, und zwar im vorliegenden Falle Nitrophenyl-β-galactosid in Tris-HCl-Puffer, pH 7.3.

Das Testprinzip beruht auf dem Ablauf folgender drei Reaktionen :

1. Der immunologischen Reaktion zwischen den an die hydrophilen Latexpartikel kovalent gebundenen $T_4$-Antikörpern einerseits und dem enzymmarkierten $T_4$ (« Enzymkonjugat ») und dem in der Probe enthaltenen freien $T_4$ andererseits. Bei dieser Reaktion findet also eine kompetitive Bindung zwischen den solid-face-Antikörpern, dem Enzymkonjugat und dem $T_4$ der Probe statt.

2. Der B/F-Trennung (B = bound, F = free). Es handelt sich hier also um eine Trennung von gebundenem (bound) und ungebundenem (free) Enzymkonjugat. Diese Trennung kann in vorteilhafter Weise durch Zentrifugieren oder durch Dialyse, beispielsweise gegen Wasser oder eine geeignete Pufferlösung, erreicht werden.

3. Der Enzymnachweisreaktion, die zwischen dem Enzymkonjugat und dem üblicherweise verwendeten, für das eingesetzte Enzym spezifischen, Substrat abläuft und die kolorimetrisch oder auf andere bekannte Weise verfolgt werden kann. Die Enzymaktivität kann entweder im Zentrifugat (free-Anteil) oder in dem resuspendierten Niederschlag (bound-Anteil) erfolgen.

Versuchsdurchführung

Die in oben angegebener Weise hergestellte Latex-anti-$T_4$-Suspension wird im Wasser oder Pufferlösung im Verhältnis 1 : 1 000 verdünnt. 0,5 ml dieser Suspension werden mit 100 μl einer $T_4$-Serum-Standardlösung mit verschiedenem, jeweils bekanntem $T_4$-Gehalt und 100 μl einer $T_4$-β-Gal-Konjugat-Lösung versetzt. Das Reaktionsgemisch wird 30 Minuten bei Raumtemperatur inkubiert. Während der Inkubation wurde ein Ansatz ständig geschüttelt, während ein zweiter Ansatz mit gleichem $T_4$-Standardgehalt lediglich stehen gelassen wurde. Danach wurde jeweils die Suspension zentrifugiert und der Überstand, in dem sich die F-Phase befindet, abgetrennt. Die B-Phase, also das an die solid-face-Antikörper gebundene $T_4$-β-Gal-Konjugat, befindet sich im Niederschlag. Zur Bestimmung der Enzymaktivität wird dieser in überschüssiger Substrat-Lösung, bestehend aus

| | |
|---|---|
| 450 mg/l | p-Nitrophenyl-β-galactosid (Fa. Sigma) |
| 100 mM | Natriumchlorid |
| 10 mM | Magnesiumchlorid |

10 mMTris-Puffer/HCl
0,4 % (V/V) Mercaptoäthanol
pH-Wert 7.3

versetzt und in bekannter Weise die Extinktion bei einer Wellenlänge von 405 nm gemessen. Es zeigte sich, daß die ohne äußere mechanische Durchmischung in der Schwebe gehaltenen Latexpartikel ein um etwa 11 % verringertes Signal ergeben.

Nachdem auf diese Weise die $T_4$-Eichkurven ermittelt wurden, wurden anschließend in derselben Weise Bestimmungen durchgeführt, bei denen anstelle von 100 µl $T_4$-Standardserum jeweils 100 µl von Proben unbekannten $T_4$-Gehalts eingesetzt und die Extinktion der B-Phasen nach der Resuspendierung in Substratlösung gemessen werden.

Es wurde festgestellt, daß die hydrophilen Latexpartikel als Träger für Antikörper und auch Antigene hervorragend geeignet sind und deren Immunreaktivität nicht beeinträchtigen. Sie können daher mit großem Vorteil bei solid-face-Enzymimmunotesten als Träger eingesetzt werden. Weiter wurde festgestellt, daß die hydrophilen Latexpartikel in Puffermilieu über lange Zeit hinweg in der Schwebe bleiben, daß die Dichte der Partikel also etwa gleich eins ist. Darüber hinaus sind die erfindungsgemäßen hydrophilen Latexpartikel gut zentrifugierbar und resuspendierbar, ohne an Immunreaktivität einzubüßen.

## Beispiel 13

Enzymimmunoassay (EIA) zur Bestimmung von Human-Thyreotropin (TSH)

Die Bestimmung von TSH in Humanserum ist von erheblicher Bedeutung für die Diagnose von Schilddrüsenerkrankungen. Eine primäre Hypothyreose erkennt man an einer stark erhöhten Basal-TSH-Konzentration (10 bis 100 µU TSH/ml). Darüber hinaus kann eine Hyperthyreose mit Sicherheit dann ausgeschlossen werden, wenn der TSH-Basalwert im Serum von 0,5 bis 3 µU/ml nach Stimulation mit TRH (Thyroidea-releasing-hormon) nicht ansteigt. Steigt der TSH-Wert dagegen um mindestens 2,5 µU/ml, jedoch um nicht mehr als 25 µU/ml, dann ist der Schilddrüsen-Stoffwechsel mit hoher Wahrscheinlichkeit ungestört. Ein Anstieg um mehr als 25 µU/ml von einem mehr oder weniger leicht erhöhten Basalwert läßt auf eine latente präklinische Hypothyreose schließen. Es sind bereits Enzymimmunoassays für TSH bekannt (Clinica Chemica Acta *67*, 263-268 (1976) ; enzyme labelled immunoassay of hormones and drugs, herausgegeben von S.B. Pal, Walter de Gruyter, Berlin und New York, 1978, S. 327-337 ; Analytical Biochemistry *96*, 419-425 (1979)). Diese bekannten Bestimmungsmethoden benötigen jedoch übermäßig lange Inkubationszeiten (bis zu 5 Tage), sind unempfindlich (5 µU TSH/ml) oder setzen aufwendige Meßgeräte voraus (Fluorometer oder Luminometer), wobei außerdem eine Vorrichtung zum Mischen der Ansätze erforderlich ist.

Die Verwendung der erfindungsgemäßen hydrophilen Latexpartikel ermöglicht nun eine photometrische Bestimmung des TSH in einer Gesamtinkubationszeit von nur 2 1/2 Tagen nach der Doppelantikörper-Trenntechnik. Dabei wird der zweite Antikörper an die erfindungsgemäßen hydrophilen Latexpartikel kovalent gebunden. Die gebundene Enzymaktivität wird dann nach einer B/F-Trennung gemessen. Da die Latexpartikel eine Dichte aufweisen, die nicht wesentlich höher als diejenige von Wasser ist, bleiben sie auch während der Enzymreaktion in der Schwebe, wodurch das übliche Mischen entfällt. Bei Verwendung von sehr verdünnten Latexsuspensionen, die aufgrund der hohen Beladbarkeit mit Antikörpern ohne weiteres möglich ist, entfällt auch das Zentrifugieren der Partikel nach Ablauf der Enzymreaktion, da durch die Suspension hindurch photometrisch gemessen werden kann.

Im Gegensatz zu den üblicherweise in immunologischen Testverfahren verwendeten Latices lassen sich die antikörperbeladenen, erfindungsgemäßen hydrophilen Polyglycidylmethacrylatpartikel nach dem Zentrifugieren leicht resuspendieren. Aus demselben Grund zeigt sich eine sehr geringe nichtspezifische Adsorption des enzymmarkierten Antigens (gebundene Aktivität in Abwesenheit vom primären Antikörper).

Versuchsdurchführung

0,2 ml TSH-Standard (internationales Referenzpräparat MRC 68/38 in TSH-freiem Serum) werden mit 0,1 ml einer 1 : 150 000 Verdünnung in Wasser oder geeigneter Pufferlösung eines an sich bekannten Anti-TSH-Antiserums, beispielsweise von Meerschweinchen, inkubiert. Nach 12 Stunden werden 0,1 ml einer Lösung eines Enzymkonjugats, bestehend aus an Glukoseoxidase (GOD) kovalent gekoppeltem TSH (entsprechend ca. $1{,}5 \times 10^{-9}$ g TSH), zu dem Gemisch der ersten Stufe pipettiert. Zu diesem Gemisch werden nach weiteren 12 Stunden 0,1 ml einer Latexsuspension pipettiert. Die Suspension enthält 0,1 bis 1 mg/ml Methacrylatlatex, wobei — wie in Beispiel 11 beschrieben — pro 1 000 mg der trockenen Latexpartikel 5 bis 150 mg Protein einer Immunglobulinfraktion, die aus einem Ziegenantiserum gegen Meerschweinchen-IgG gewonnen wurde, hinzugefügt und so an die hydrophilen Latexpartikel kovalent gebunden worden sind. Nach einer Stunde wird zentrifugiert und der Niederschlag der Latexpartikel mit 1 ml einer geeigneten Pufferlösung gewaschen, wodurch die Latexpartikel resuspendiert werden. Anschließend wird erneut mehrfach zentrifugiert und gewaschen. Die Überstände werden verworfen.

Zu jedem Ansatz wird 1 ml einer Substratlösung für die GOD pipettiert und kurz geschüttelt. Die Latexpartikel bleiben danach mindestens 2 Stunden lang gleichmäßig suspendiert. Danach wird die Suspension in eine Meßküvette überführt und die Extinktion bei 405 nm gemessen. Von jeder Extinktion wird ein Substratleerwert $L_1$ abgezogen. $L_1$ ist die Extinktion von 1 ml Substratlösung, die die gleiche Masse beladener Latexpartikel enthält wie die oben erwähnte Latex-Suspension, die zu dem Gemisch aus Anti-TSH-Antiserum und TSH-GOD zugegeben wurde.

Die so ermittelten Extinktionen werden für verschiedene Standardkonzentrationen an TSH aufgezeichnet. Mit Hilfe dieser Eichkurven werden die Konzentrationen von TSH in klinischen Proben unbekannten Gehaltes anhand der ermittelten Extinktionen abgelesen.

Als geeingnete Pufferlösung wird vorzugsweise eine 0,04 M Phosphat-Lösung, pH 7, 4, verwendet, die 0,005 M EDTA, 0,1 % Rinderserumalbumin sowie 0,15 M NaCl enthält.

Als GOD-Substratlösung wird bevorzugt eine wäßrige Lösung eingesetzt, die 5 g/100 ml Glukose, 100 mg/100 ml 2,2'-Azino-di-[3-ethyl-benzthiazolon-sulfonsäure (6)] (ABTS), 5 mg/100 ml Peroxidase (POD), 0,05 M Phosphatpuffer, pH 5,6, enthält.

Zum Vergleich wurde der TSH-Gehalt dreier Serumproben sowohl mit dem vorstehend beschriebenen EIA (A) als auch mit Hilfe eines bekannten Doppelantikörper-RIA (B) bestimmt. Wie die folgende Tabelle zeigt, stimmen die jeweils ermittelten Werte gut überein :

| | (A) | (B) |
|---|---|---|
| Serum 1 | 11,2 µU/ml | 12,5 µU/ml |
| Serum 2 | 7,3 µU/ml | 7,1 µU/ml |
| Serum 3 | 3,8 µU/ml | 4,5 µU/ml |

Auch aus der vorstehend beschriebenen TSH-Bestimmung ergibt sich, daß die erfindungsgemäßen hydrophilen Latexpartikel als Träger für biologisch und/oder immunologisch aktive Substanzen, insbesondere für Antikörper hervorragend geeignet sind.

Ermittlung der unspezifischen Bindung

Eine TSH-Standard-Probe wird in der oben unter « Versuchsdurchführung » beschriebenen Weise behandelt (Probe C) und mit einer weiteren gleichartigen Probe (Probe D) verglichen, die nahezu den identischen Umsetzungen wie Probe C unerworten worden ist. Es wurde lediglich das Anti-TSH-Antiserum weggelassen. Der Vergleich der für die beiden Proben C und D in der Suspension gemessenen Extinktionen

| | Probe C | Probe D |
|---|---|---|
| Extinktion gemessen in der Suspension abzüglich $L_1$ | 1,028 | 0,015 |

zeigt, daß die unspezifische Bindung weniger als 1,5 % beträgt.

**Patentansprüche**

1. Aus einem Homo- oder Copolymerisat von in Wasser schwerlöslichen Monomeren bestehende hydrophile Latexpartikel, dadurch gekennzeichnet, daß sie durch Emulsionspolymerisation in Anwesenheit eines wasserlöslichen, Radikale bildenden Initiators, jedoch ohne jeden Zusatz eines Emulgators, Stabilisators oder Netzmittels herstellbar sind und daß das Monomer (im Falle eines Homopolymerisats) bzw. ein Teil der Monomeren (im Falle eines Copolymerisats) ein mindestens eine polymerisierbare C = C-Doppelbindung in Molekül enthaltendes Epoxid ist.

2. Hydrophile Latexpartikel nach Anspruch 1, dadurch gekennzeichnet, daß das Monomer bzw. ein Teil der Monomeren eine Epoxy-alkylen-Verbindung bzw. ein Glycidester oder Glycidyläther ist.

3. Hydrophile Latexpartikel nach Anspruch 2, dadurch gekennzeichnet, daß das Monomer bzw. ein Teil der Monomeren Glycidylacrylat oder Glycidylmethacrylat ist.

4. Hydrophile Latexpartikel nach einem der Ansprüche 1 bis 3, dadurch gekennzeichnet, daß das Homo- oder Copolymerisat mit Hilfe eines zwei- oder mehrfach ungesättigten Vernetzungsmittels vernetzt ist.

5. Hydrophile Latexpartikel nach einem der Ansprüche 1 bis 4, dadurch gekennzeichnet, daß die Teilchen monodisperse Kügelchen sind und einen untereinander nahezu gleich großen Durchmesser von zwischen 0,15 und 1,5 µm besitzen.

6. Hydrophile Latexpartikel nach einem der Ansprüche 1 bis 5, dadurch gekennzeichnet, daß das Homo- oder Copolymerisat endständige Hydroxyl-, primäre oder sekundäre Amino-, Thiol-, Aldehyd- oder Carboxylgruppen aufweist.

7. Hydrophile Latexpartikel nach einem der Ansprüche 1 bis 6, dadurch gekennzeichnet, daß sie einpolymerisierte Farbstoffe oder fluoreszierende Verbindungen enthalten.

8. Verfahren zur Herstellung der hydrophilen Latexpartikel gemäß einem der Ansprüche 1 bis 7, dadurch gekennzeichnet, daß ein in Wasser schwerlösliches Monomer oder verschiedene in Wasser schwerlösliche Monomere, wobei das Monomer bzw. ein Teil der Monomeren ein mindestens eine polymerisierbare C = C-Doppelbindung im Molekül enthaltendes Epoxid ist, in Wasser dispergiert und unter Ausschluß von Sauerstoff, beispielsweise in einer Inertgasatmosphäre, durch Emulsionspolymerisation in Anwesenheit eines wasserlöslichen, Radikale bildenden Initiators, jedoch ohne jeden Zusatz eines Emulgators, Stabilisators oder Netzmittels homo- oder copolymerisiert werden.

9. Verfahren nach Anspruch 8, dadurch gekennzeichnet, daß ein mindestens eine (co)polymerisierbare C = C-Doppelbindung im Molekül enthaltendes Epoxid, gegebenenfalls im Gemisch mit anderen copolymerisierbaren Monomeren, in Wasser dispergiert und durch Emulsionspolymerisation ohne Zusatz eines Emulgators homo- oder copolymerisiert wird.

10. Verfahren nach Anspruch 9, dadurch gekennzeichnet, daß als Epoxid eine Epoxy-alkylen-Verbindung bzw. ein Glycidylester oder Glycidyläther verwendet wird.

11. Verfahren nach Anspruch 9 oder 10, dadurch gekennzeichnet, daß als Epoxid Glycidylacrylat oder Glycidylmethacrylat verwendet wird.

12. Verfahren nach einem der Ansprüche 8 bis 11, dadurch gekennzeichnet, daß die Emulsionspolymerisation unter Zusatz eines zwei- oder mehrfach ungesättigten Vernetzungsmittels durchgeführt wird.

13. Verfahren nach einem der Ansprüche 8 bis 12, dadurch gekennzeichnet, daß bei der Emulsionspolymerisation das Flottenverhältnis (Wasser : Monomerenphase) 8 : 1 bis 16 : 1, bezogen auf die Volumina, beträgt.

14. Verfahren nach einem der Ansprüche 8 bis 13, dadurch gekennzeichnet, daß die Initiatorkonzentration in der wäßrigen Phase 0,5 bis 1,5 g/l beträgt.

15. Verfahren nach einem der Ansprüche 9 bis 14, dadurch gekennzeichnet, daß die Konzentration des Epoxids in der Monomerenphase 1 bis 100 Gew.% beträgt.

16. Verfahren nach einem der Ansprüche 8 bis 15, dadurch gekennzeichnet, daß die Emulsionspolymerisation bei einer Temperatur von zwischen 0° und 80 °C durchgeführt wird.

17. Verfahren nach einem der Ansprüche 8 bis 16, dadurch gekennzeichnet, daß der endständige Epoxid-Gruppen enthaltende Latex noch während oder nach der Emulsionspolymerisation mit wäßriger Alkalihydroxidlösung, wäßriger Ammoniaklösung, mit einem primären Amin, einem Hydrazin oder einem Sulfid, einer Hydrolyse, Ammonolyse, Aminolyse oder Thiolyse unterworfen wird, so daß sich endständige Hydroxyl-, gegebenenfalls mono- oder disubstituierte Aminogruppen oder Thiolgruppen bilden, welche gegebenenfalls anschließend enzymatisch oder mit Periodat bzw. Periodsäure in Aldehydgruppen übergeführt werden.

18. Diagnostisches Mittel, enthaltend hydrophile Latexpartikel gemäß einem der Ansprüche 1 bis 7 als Träger und an diesen Träger direkt oder über ein Kupplungsmittel als « Brücke » kovalent gebundene biologisch und/oder immunologisch aktive Substanzen.

19. Diagnostisches Mittel gemäß Anspruch 18, dadurch gekennzeichnet, daß es als biologisch und/oder immunologisch aktive Substanzen Peptide, Proteine, Enzyme, Hormone, Vitamine, Antigene, Antikörper oder Mikroorganismen enthält.

20. Diagnostisches Mittel gemäß Anspruch 19 zur Bestimmung von Thyroxin, dadurch gekennzeichnet, daß es als biologisch und/oder immunologisch aktive Substanz einen Thyroxin-Antikörper enthält.

21. Diagnostisches Mittel gemäß Anspruch 19 zur Bestimmung von Human-Thyreotropin, dadurch gekennzeichnet, daß es als biologisch und/oder immunologisch aktive Substanz einen Human-Thyreotropin-Antikörper enthält.

**Claims**

1. Hydrophilic latex particles consisting of a homo- or co-polymer of monomers which are sparingly soluble in water, characterised in that they can be prepared by emulsion polymerisation in the presence of a water-soluble, radical-forming initiator but without any addition of an emulsifier, stabiliser or wetting agent and that the monomer (in the case of a homo-polymer) or a part of the monomers (in the case of a co-polymer) is an epoxide containing at least one polymerisable C = C double bond in the molecule.

2. Hydrophilic latex particles according to claim 1, characterised in that the monomer or a part of the monomers is an epoxyalkylene compound or a glycide ester or glycidyl ether.

3. Hydrophilic latex particles according to claim 2, characterised in that the monomer or a part of the monomers is glycidyl acrylate or glycidyl methacrylate.

4. Hydrophilic latex particles according to one of claims 1 to 3, characterised in that the homo- or copolymer is cross-linked with the help of a di- or polyunsaturated cross-linking agent.

5. Hydrophilic latex particles according to one of claims 1 to 4, characterised in that the particles are monodisperse spheroids amongst each other possessing an almost equally large diameter of between 0.15 and 1.5 μm.

6. Hydrophilic latex particles according to one of claims 1 to 5, characterised in that the homo- or copolymer has terminal hydroxyl, primary or secondary amino, thiol, aldehyde or carboxyl groups.

7. Hydrophilic latex particles according to one of claims 1 to 6, characterised in that they contain polymerised-in dyestuffs or fluorescing compounds.

8. Process for the preparation of hydrophilic latex particles according to one of claims 1 to 7, characterised in that a monomer which is sparingly soluble in water or various monomers which are sparingly soluble in water, whereby the monomer or a part of the monomers is an epoxide containing at least one polymerisable C = C double bond in the molecule, are dispersed in water and, with the exclusion of oxygen, for example in an inert atmosphere, are homo- or co-polymerised by emulsion polymerisation in the presence of a water-soluble, radical-forming initiator but without any addition of an emulsifier, stabiliser or wetting agent.

9. Process according to claim 8, characterised in that an epoxide containing at least one (co)polymerisable C = C double bond in the molecule is dispersed in water, possibly in admixture with other co-polymerisable monomers, and is homo- or co-polymerised by emulsion polymerisation without the addition of an emulsifier.

10. Process according to claim 9, characterised in that an epoxyalkylene compound or a glycidyl ester or glycidyl ether is used as the epoxide.

11. Process according to claim 9 or 10, characterised in that glycidyl acrylate or glycidyl methacrylate is used as epoxide.

12. Process according to one of claims 8 to 11, characterised in that the emulsion polymerisation is carried out with the addition of a di- or polyunsaturated cross-linking agent.

13. Process according to one of claims 8 to 12, characterised in that in the case of the emulsion polymerisation, the bath ratio (water : monomer phase) amounts to 8 : 1 to 16 : 1, referred to the volumes.

14. Process according to one of claims 8 to 13, characterised in that the initiator concentration in the aqueous phase amounts to 0.5 to 1/5 g/litre.

15. Process according to one of claims 9 to 14, characterised in that the concentration of the epoxide in the monomer phase amounts to 1 to 100 % by weight.

16. Process according to one of claims 8 to 15, characterised in that the emulsion polymerisation is carried out at a temperature of between 0° and 80 °C.

17. Process according to one of claims 8 to 16, characterised in that the latex containing terminal epoxide groups is also subjected, during or after the emulsion polymerisation, to a hydrolysis, ammonolysis, aminolysis or thiolysis with aqueous alkali metal hydroxide solution, aqueous ammonia solution, with a primary amine, a hydrazine or a sulphide so that terminal hydroxyl, possibly mono- or disubstituted amino groups or thiol groups are formed which are possibly subsequently converted into aldehyde groups enzymatically or with periodate or periodic acid.

18. Diagnostic agent containing hydrophilic latex particles according to one of claims 1 to 7 as a carrier and biologically and/or immunologically active substances covalently bound to this carrier directly or *via* a coupling agent as a « bridge ».

19. Diagnostic agent according to claim 18, characterised in that, as biologically and/or immunologically active substances, it contains peptides, proteins, enzymes, hormones, vitamins, antigens, antibodies or micro-organisms.

20. Diagnostic agent according to claim 19 for the determination of thyroxine, characterised in that it contains a thyroxine antibody as biologically and/or immunologically active substance.

21. Diagnostic agent according to claim 19 for the determination of human thyreotropin, characterised in that it contains a human thyreotropin antibody as biologically and/or immunologically active substance.

**Revendications**

1. Particules de latex hydrophiles constituées par un homopolymère ou un copolymère obtenu à partir de monomères difficilement solubles dans l'eau, caractérisées en ce qu'elles peuvent être obtenues par polymérisation en émulsion en présence d'un initiateur hydrosoluble formateur de radicaux, mais sans l'addition d'aucun agent émulsifiant, stabilisant ou mouillant et en ce que le monomère (dans le cas d'un homopolymère) ou une partie des monomères (dans le cas d'un copolymère) est un époxyde ayant dans sa molécule au moins une double liaison C = C polymérisable.

2. Particules de latex hydrophiles selon la revendication 1, caractérisées en ce que le monomère ou une partie des monomères est un composé du type époxy-alkylène ou bien il est un ester glycidique ou un éther glycidique.

3. Particules de latex hydrophiles selon la revendication 2, caractérisées en ce que le monomère ou une partie des monomères est l'acrylate glycidique ou le méthacrylate glycique.

4. Particules de latex hydrophiles selon l'une quelconque des revendications 1 à 3, caractérisées en ce que l'homopolymère ou le copolymère est réticulé à l'aide d'un agent réticulant deux ou plusieurs fois insaturé.

5. Particules de latex hydrophiles selon l'une quelconque des revendications 1 à 4, caractérisées en ce que les particules sont des globules monodispersés présentant un diamètre très peu variable d'un globule à l'autre, compris entre 0,15 et 1,5 μm.

6. Particules de latex hydrophiles selon l'une quelconque des revendications 1 à 5, caractérisées en ce que l'homopolymère ou le copolymère comporte des groupes terminaux hydroxyle, des groupes amino primaires ou secondaires, ou des groupes thiol, aldéhyde ou carboxyle.

7. Particules de latex hydrophiles selon l'une quelconque des revendications 1 à 6, caractérisées en ce qu'elles comportent des colorants ou des composés fluorescents incorporés par polymérisation.

8. Procédé de fabrication de particules de latex hydrophiles selon l'une quelconque des revendications 1 à 7, caractérisé en ce qu'on disperse dans l'eau un monomère difficilement soluble dans l'eau ou plusieurs monomères difficilement solubles dans l'eau, le monomère ou une partie des monomères étant un époxyde comportant dans sa molécule au moins une double liaison C = C polymérisable, que l'on soumet à l'exclusion de l'oxygène, par exemple sous l'atmosphère d'un gaz inerte, à une homopolymérisation ou une copolymérisation, par polymérisation en émulsion en présence d'un initiateur hydrosoluble formateur de radicaux, sans addition toutefois d'aucun agent émulsifiant, stabilisant ou mouillant.

9. Procédé selon la revendication 8, caractérisé en ce qu'on disperse dans l'eau un époxyde comportant dans sa molécule au moins une double liaison C = C polymérisable, éventuellement mélangé à d'autres monomères copolymérisables, et on le soumet à une polymérisation en émulsion ou à une copolymérisation en émulsion sans l'addition d'un émulsifiant.

10. Procédé selon la revendication 9, caractérisé en ce qu'on utilise comme époxyde un composé du type époxy-alkylène ou bien un ester glycidique ou un éther glycidique.

11. Procédé selon la revendication 9 ou 10, caractérisé en ce qu'on utilise comme époxyde l'acrylate glycidique ou le méthacrylate glycidique.

12. Procédé selon l'une quelconque des revendications 8 à 11, caractérisé en ce que la polymérisation en émulsion est effectuée en ajoutant un agent réticulant deux ou plusieurs fois insaturé.

13. Procédé selon l'une quelconque des revendications 8 à 12, caractérisé en ce qu'au cours de la polymérisation en émulsion le rapport de volume entre les liquides (eau/phase des monomères) est compris entre 8/1 et 16/1.

14. Procédé selon l'une quelconque des revendications 8 à 13, caractérisé en ce que la concentration de l'initiateur dans la phase aqueuse est comprise entre 0,5 et 1,5 g/l.

15. Procédé selon l'une quelconque des revendications 9 à 14, caractérisé en ce que la concentration de l'époxyde dans la phase des monomères est comprise entre 1 et 100 % en poids.

16. Procédé selon l'une quelconque des revendications 8 à 15, caractérisé en ce que la polymérisation en émulsion est conduite à une température comprise entre 0 et 80 °C.

17. Procédé selon l'une quelconque des revendications 8 à 16, caractérisé en ce que le latex comportant des groupes époxyde terminaux est soumis encore pendant ou bien après la polymérisation en émulsion à une hydrolyse, ammonolyse, aminolyse ou thiolyse au moyen d'une solution aqueuse d'un hydroxyde alcalin, d'ammoniaque, d'une amine primaire, d'une hydrazine ou d'un sulfure, de sorte qu'il se forme des groupes terminaux hydroxyle, amino portant éventuellement un ou deux substituants, ou thiol, que l'on transforme éventuellement ensuite par voie enzymatique ou à l'aide de periodate ou d'acide periodique en groupes aldéhyde.

18. Agent de diagnostic, renfermant des particules de latex hydrophiles selon l'une quelconque des revendications 1 à 7 comme supports ainsi que, liées par covalence à ces supports, directement ou au moyen d'un agent de couplage en tant que « pont », des substances ayant une activité biologique et/ou immunologique.

19. Agent de diagnostic selon la revendication 18, caractérisé en ce qu'il comporte comme substances ayant une activité biologique et/ou immunologique, des peptides, des protéines, des enzymes, des hormones, des vitamines, des antigènes, des anticorps ou des micro-organismes.

20. Agent de diagnostic selon la revendication 19 pour le dosage de la thyroxine, caractérisé en ce qu'il comporte comme substance ayant une activité biologique et/ou immunologique un anticorps de la thyroxine.

21. Agent de diagnostic selon la revendication 19 pour le dosage de la thyréotropine humaine, caractérisé en ce qu'il comporte comme substance ayant une activité biologique et/ou immunologique, un anticorps de la thyréotropine humaine.